# EUROPEAN PATENT SPECIFICATION

(11) **EP 0 477 197 B1**
(45) Date of publication and mention of the grant of the patent: **12.07.1995**
(21) Application number: 90907684.6
(22) Date of filing: 15.05.1990
(51) Int. Cl.: C07D 277/593

(54) **PREPARATION OF 2-(2-TRITYLAMINOTHIAZOL-4-YL)-2-SYN-METHOXYIMINOACETIC ACID**
HERSTELLUNG VON 2-(2-TRITYLAMINOTHIAZOL-4-YL)-2-SYN-METHOXYIMINOESSIGSÄURE
PREPARATION D'ACIDE 2-(2-TRITYLAMINOTHIAZOL-4-YL)-2-SYN-METHOXYAMINOACETIQUE

(30) Priority: 12.06.1989 US 365450
(43) Date of publication of application: 01.04.1992
(73) Proprietor: THE UPJOHN COMPANY, Kalamazoo, Michigan 49001 (US)
(72) Inventor: SHEPHARD, Kenneth, Paul, Kalamazoo, MI 49002 (US)
(74) Representative: Perry, Robert Edward
(86) International application number: US9002620
(87) International publication number: WO9015802

(56) References cited:
- US-A- 4 376 203
- CHEMICAL ABSTRACTS, Vol. 110, No. 7, 13 February 1989, Columbus, Ohio, US, page 662, abstract 57407g, & CS, A, 246443
- CHEMICAL ABSTRACTS, Vol. 110, No. 7, 13 February 1989, Columbus, Ohio, US, page 691, abstract 57653j, & CS, A. 245873

## Description

### BACKGROUND OF THE INVENTION

### 1. Field of the Invention

The process of the present invention is the conversion of an aminothiazole to the corresponding thiazole acetic acid which is a precursor in the preparation of cephalosporin antibiotics.

### 2. Description of the Related Art

2-(2-Tritylaminothiazol-4-yl)-2-syn-methoxyiminoacetic acid is a known compound (US Patent 4,376,203, Example 3, Step C) useful in the production of a cephalosporin antibiotic, CEFTIOFUR (ceftiofur sodium, US Patent 4,464,367).

GB Patent 1,580,621 (Example 21, Stages C-F) and US Patent 4,376,203 (Example 21, Steps C-F) set forth a process the transformation of ethyl 2-(2-aminothiazol-4-yl)-2-syn-hydroxyiminoacetic acid to 2-(2-tritylaminothiazol-4-yl)-2-syn-methoxyiminoacetic acid, see Stages D-F.

Chemical Abstracts 110:57407g of December 15, 1987 reports the conversion of ethyl 2-(2-aminothazol-4-yl)-2-syn-methoxyiminoacetic acid to ethyl 2-(2-tritylaminothiazol-4-yl)-2-syn-methoxyiminoacetic acid by reacting the 2-aminothiazole with 0.8-1.6 mole φ₃C-Cl in the presence of a strong inorganic base at room temperature.

Chemical Abstracts 110:57653j of December 15, 1987 reports the conversion of ethyl 2-(2-aminothazol-4-yl)-2-syn-methoxyiminoacetic acid to ethyl 2-(2-tritylaminothiazol-4-yl)-2-syn-methoxyiminoacetic acid by reacting the 2-aminothiazole with ClCH₂-CO-Cl in the presence of an inorganic base at room temperature.

The process of the present invention is superior to the known methods of producing the thiazole acetic acid (IV) because none of the intermediates are isolated. Up to this time either the thiazole ester (II) or thiazole salt (III) or both were isolated, leading to a long process. The one-pot process of the invention can easily be accomplished, without any solvent changes, in one day giving the product in a higher yield.

### SUMMARY OF INVENTION

Disclosed is a process for the preparation of a thiazole acetic acid of formula (IV)
where R₂ is -H,
C₁-C₄ alkyl,
-CO-NH-CH₃,
-CH₂-CO-CH₂,
1,2,4-triazol-5-ylmethyl,
1,2,3-triazol-5-ylmethyl,
-CH₂-CO-OCH₃,
-CH₂-COOH,
-C(R₂₋₁)(R₂₋₂)-CO-O-R₂₋₃ where R₂₋₁ and R₂₋₂ are -H or -CH₃ and R₂₋₃ is C₁-C₄ alkyl, or
-C(R₂₋₁)(R₂₋₂)-COOH where R₂₋₁ and R₂₋₂ are as defined above; and
R₃ is φ₃C- or Cl₂CH-CO-, which comprises
(1) contacting an aminothiazole of formula (I) where R₁ is C₁-C₄ alkyl, benzyl or p-methoxybenzyl and where R₂ is as defined above, with a compound of the formula R₃-X₁ where X₁ is -Cl or -Br and where R₃ is as defined above, in the presence of a non-aqueous base,
(2) hydrolyzing the product of step (1) with aqueous base, in the presence of a solvent for the hydrolysis product, and
(3) acidifying to a pH < 7, without isolation of any intermediates.

### DETAILED DESCRIPTION OF THE INVENTION

While there are a number of procedures for the transformation of ethyl 2-(2-aminothazol-4-yl)-2-syn-methoxyiminoacetic acid to 2-(2-tritylaminothiazol-4-yl)-2-syn-methoxyiminoacetic acid, the process of the present invention is a one-pot process which gives improved yields.

The aminothiazole (I) is contacted with the desired alkylating agent R₃-X₁ in an organic solvent in the presence of a non-aqueous base. The particular R₂ group utilized will be chosen because of the particular R₂ desired in the final product (cephalosporin) of which the thiazole acetic acid (IV) is a portion. For example, if one desires to produce ceftiofur sodium (US Patent 4,464,367) then it is preferred that R2 is methyl. X₁ is -Cl or -Br. The non-aqueous base can be either an organic base or an inorganic base. Suitable organic bases include pyridine optionally substituted with 1-3 -CH₃, DBU, DBN, NX₃X₄X₅ where X₃, X₄ and X₅ are the same or different and are C₁-C₄ alkyl and where two of X₃, X₄ and X₅ are taken together with the attached nitrogen atom to form a compound selected from pyrrolidine, piperidine, morpholine or piperazine, and the other of X₃, X₄ and X₅ is as defined above. Preferred organic bases include pyridine and triethylamine; more preferred is pyridine. The organic base can serve as the solvent for the reaction. Suitable inorganic bases include bicarbonate (HCO₃⁻¹) and carbonate (CO₃⁻²). Other suitable solvents include THF, N-methylpyrrolidinone, DMAC and acetone. The reaction is operable in a temperature range of about 20-100°, preferably at about 20-50°. The tritylation step is complete within about 1 hr at about 60-70° using 1.1 equivalents of trityl chloride and 5 ml of pyridine/g of reactants. During the tritylation reaction about 1-2% of the anti oxime is formed. At about 40° the reaction is still complete and no anti oxime is detected.

The product of the above step is then hydrolyzed with aqueous base. Any aqueous base which will cleave the ester is operable. Suitable aqueous bases include hydroxides, alkoxides of the formula O-X₂⁻¹ where X₂ is C₁-C₄ alkyl, and equivalents thereof. Preferred aqueous bases include hydroxides; more preferred are sodium or potassium hydroxide. About 3-4 equivalents of the aqueous base is preferred. If less is used the reaction rate is slowed, if more is used no advantage is observed. The hydrolysis is complete in about 3 hr at about 60° and in about 2 hr at about 70°. The temperature is not important to the yield, it only affects the rate of the reaction. Operable temperatures are from about 20-100°, preferably from about 50 to about 80°. The temperature used is affected by the amount of solvent present. With pyridine, the more solvent used the faster the reaction rate at a given temperature.

Following hydrolysis, the reaction mixture is acidified to a pH of about 2 to form the free acid. Any acid sufficiently strong is operable as is well known to those in the art. Operable acids include sulfuric, hydrochloric, phosphoric, nitric, trifluoroacetic, perchloric and equivalents thereof; preferred are sulfuric or hydrochloric. Prior to acidifying it is preferred to dilute the reaction mixture somewhat with an organic solvent such as methylene chloride. If this step is performed in less than one hr, very little of the anti oxime is formed. However, if this two phase system (organic solvent/water) is stirred for a long time at elevated temperatures the anti oxime forms in increasing amounts. Therefore, it is preferred to cool the thiazole salt (III) prior to acidifying and allowing the heat of neutralization bring the reaction temperature to about 30-35°. At this time the layers are separated. The organic layer is washed to remove the salt of the amine base and is then worked up by known means.

For purposes of work-up the reaction mixture can be diluted with water or an organic solvent (depending on the method of work-up desired) and the thiazole acetic acid (IV) either extracted or filtered.

The anti oxime is efficiently removed by crystallization from methylene chloride or a methylene chloride/hexane mixture. It is desired to remove the anti oxime because it is very poorly active in the cephalosporin antibiotics of which the thiazole acetic acid (IV) is a part.

The thiazole acetic acids (IV) are useful intermediates in the production of cephalosporin antibiotics. More particularly, 2-(2-tritylaminothiazol-4-yl)-2-syn-methoxyiminoacetic acid (IV) is useful in the production of ceftiofur sodium (US Patent 4,464,367).

### DEFINITIONS AND CONVENTIONS

The definitions and explanations below are for the terms as used throughout this entire document including both the specification and the claims.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" or "Rᵢ" where "i" is an integer.

Chemical formulas or portions thereof drawn in a linear fashion represent atoms in a linear chain. The symbol "-" in general represents a bond between two atoms in the chain. Thus CH₃-0-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

### II. DEFINITIONS

All temperatures are in degrees Centigrade.

TLC refers to thin-layer chromatography.

THF refers to tetrahydrofuran.

DBU refers to 1,8-diazabicyclo[5.4.0]undec-7-ene.

DBN refers to 1,5-diazabicyclo[4.3.0]non-5-ene.

DMAC refers to dimethylacetamide.

φ refers to phenyl (C₆H₅).

M includes lithium, sodium and potassium.

### EXAMPLES

### EXAMPLE 1 2-(Tritylamino)-α-(methoxyimino)-4-thiazoleacetic acid (IV)

A mixture of ethyl 2-amino-α-(methoxyimino)-4-thiazoleacetate (10.0 g), trityl chloride (13.99 g), and pryidine (20.0 ml) is stirred under nitrogen at 40°. After stirring for 1 hr the reaction is checked by TLC. When the tritylation is complete, water (20.0 ml) and aqueous sodium hydroxide (50%, 8.14 ml) are added and the mixture heated at 65° for 4-5 hr. When the hydrolysis is complete (TLC) the resulting slurry is cooled to 20° and methylene chloride (100 ml) is added. This mixture is then acidified by the addition of concentrated aqueous hydrochloric acid. The layers are separated. The organic layer is washed with water (2 x 70 ml). Heptane (150 ml) is added to the organic phase with stirring. The resulting slurry is cooled to 0° and filtered. The solids are washed with heptane/methylene chloride mixture (60/40, 200 ml) and dried under vacuum at 55° to give the title compound.

### EXAMPLE 2 2-(Tritylamino)-α(methoxyimino)-4-thiazoleacetic acid (IV)

A mixture of ethyl 2-amino-α-(methoxyimino)-4-thiazoleacetate (10.0 g), dimethylacetamide (50 ml), trityl chloride (14.59 g), and triethylamine (9.12 ml) are stirred under nitrogen at 40°. When the tritylation is complete by TLC, water (20 ml) and aqueous sodium hydroxide (50%, 8 ml) are added. This mixture is heated at 65° for 2 to 2.5 hours. When the hydrolysis is complete (TLC) the resulting slurry is cooled in an ice bath and diluted with water (50 ml). This mixture is then acidified by the addition of concentrated aqueous hydrochloric acid. This slurry is filtered. The solids are washed with water and dried under vacuum. The crude solids are stirred with a mixture of heptane/methylene chloride (70/30, 77 ml). After stirring for 30 min the slurry is filtered. The solids are washed with a mixture of heptane/methylene chloride (70/30, 150 ml) and dried under vacuum at 55° to give the title compound.

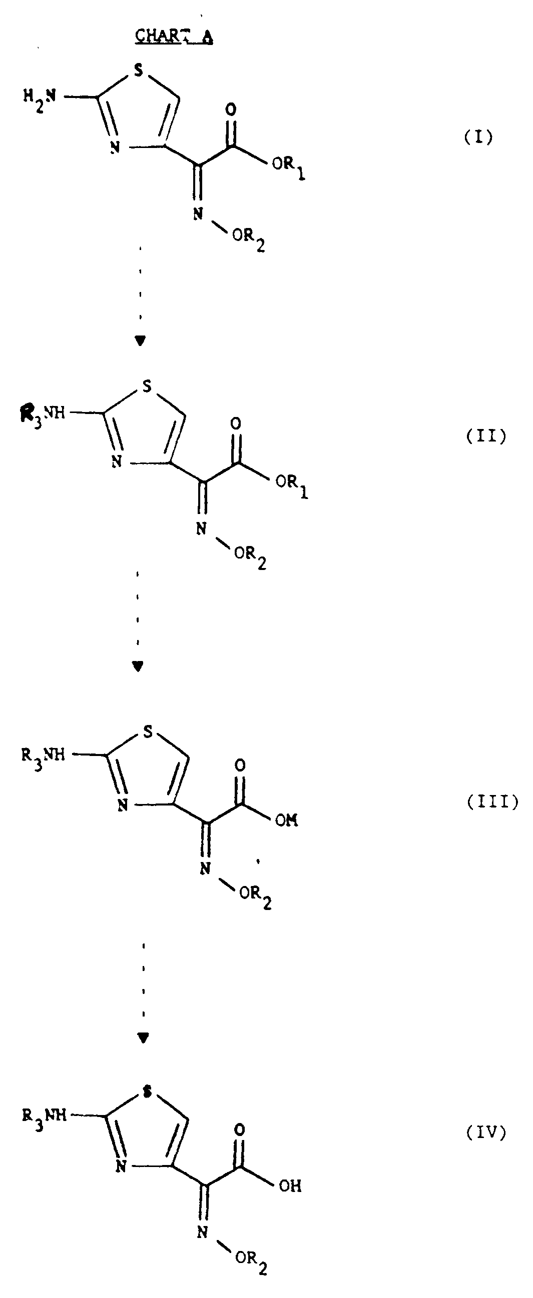

## Claims

1. A process for the preparation of a thiazole acetic acid of formula (IV) 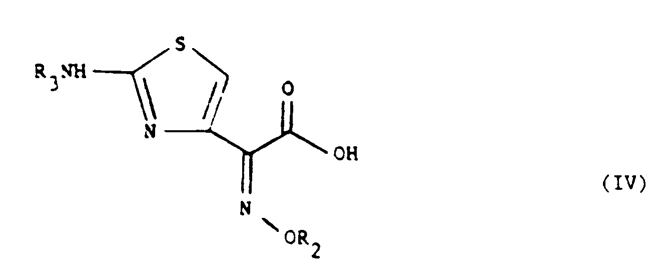 where
R₂ is -H,
C₁-C₄ alkyl,
-CO-NH-CH₃,
-CH₂-CO-CH₂,
1,2,4-triazol-5-ylmethyl,
1,2,3-triazol-5-ylmethyl,
-CH₂-CO-OCH₃,
-CH₂-COOH,
-C(R₂₋₁)(R₂₋₂)-CO-O-R₂₋₃ where R₂₋₁ and R₂₋₂ are -H or -CH₃ and R₂₋₃ is C₁-C₄ alkyl, or
-C(R₂₋₁)(R₂₋₂)-COOH where R₂₋₁ and R₂₋₂ are as defined above; and
R₃ is φ₃C- or Cl₂CH-CO-, which comprises
(1) contacting an aminothiazole of formula (I) 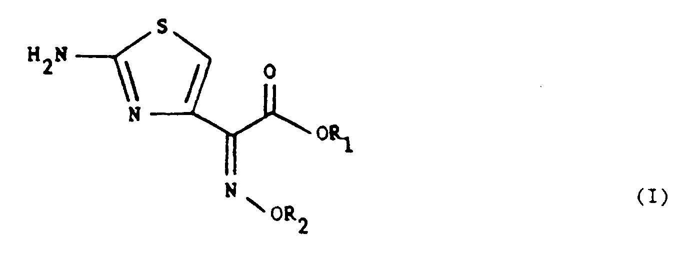 where R₁ is C₁-C₄ alkyl, benzyl or p-methoxybenzyl and where R₂ is as defined above, with a compound of the formula R₃-X₁ where X₁ is -Cl or -Br and where R₃ is as defined above, in the presence of a non-aqueous base,
(2) hydrolyzing the product of step (1) with aqueous base, in the presence of a solvent for the hydrolysis product, and
(3) acidifying to a pH < 7, without isolation of any intermediates.

2. A process according to claim 1, wherein the non-aqueous base is pyridine optionally substituted with 1-3 CH₃ groups, DBU, DBN, a tri(C₁₋₄ alkyl)amine, a N-(C₁₋₄ alkyl)pyrrolidine, a N-(C₁₋₄ alkyl)piperidine, a N-(C₁₋₄ alkyl)morpholine or a N-(C₁₋₄ alkyl)piperazine.

3. A process according to claim 2, wherein the non-aqueous base is pyridine or triethylamine.

4. A process according to claim 1, wherein the non-aqueous base comprises bicarbonate or carbonate ion.

5. A process according to any preceding claim, wherein the aqueous base comprises a hydroxide or C₁₋₄ alkoxide.

6. A process according to any preceding claim, wherein the acid is selected from sulphuric, hydrochloric, phosphoric, nitric, trifluoroacetic and perchloric acids.

7. A process according to claim 6, wherein the acid is sulphuric or hydrochloric acid.

8. A process according to any preceding claim, wherein the thiazoleacetic acid is 2-(tritylamino)-α-(methoxyimino)-4-thiazoleacetic acid.

## Patentansprüche

1. Verfahren zur Herstellung einer Thiazolessigsäure der Formel (IV) 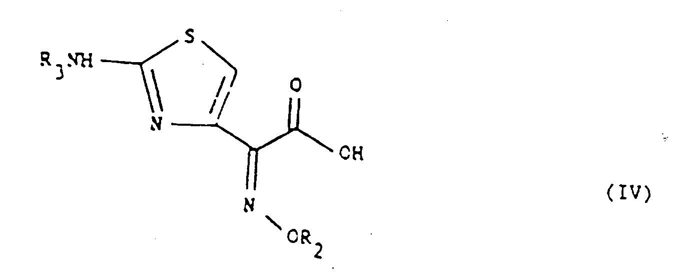 worin bedeuten:
R₂ -H,
C₁-C₄-Alkyl,
-CO-NH-CH₃,
-CH₂-CO-CH₂,
1,2,4-Triazol-5-ylmethyl,
1,2,3-Triazol-5-ylmethyl,
-CH₂-CO-OCH₃,
-CH₂-COOH,
-C(R₂₋₁)(R₂₋₂)-CO-OR₂₋₃ mit R₂₋₁ und R₂₋₂ gleich
-H oder -CH₃ und R₂₋₃ gleich C₁-C₄-Alkyl oder -C(R₂₋₁)(R₂₋₂)-COOH mit R₂₋₁ und R₂₋₂ in der zuvor angegebenen Bedeutung und
R₃ gleich φ₃C- oder Cl₂CH-CO-, durch
(1) In-Berührung-bringen eines Aminothiazols der Formel (I) 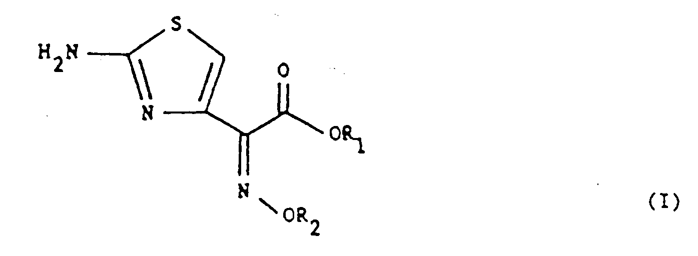 worin R₁ für C₁-C₄-Alkyl, Benzyl oder p-Methoxybenzyl steht und R₂ die zuvor angegebene Bedeutung besitzt, mit einer Verbindung der Formel R₃-X₁, worin X₁ für -Cl oder -Br steht und R₃ die zuvor angegebene Bedeutung besitzt, in Gegenwart einer nicht-wäßrigen Base,
(2) Hydrolysieren des Produkts aus Stufe (1) mit einer wäßrigen Base in Gegenwart eines Lösungsmittels für das Hydrolyseprodukt und
(3) Ansäuern auf einen pH-Wert < 7, ohne Isolieren irgendwelcher Zwischenprodukte.

2. Verfahren nach Anspruch 1, wobei die nicht-wäßrige Base aus Pyridin, gegebenenfalls substituiert durch 1-3 CH₃-Gruppe(n), DBU, DBN, einem Tri(C₁₋₄-alkyl)amin, einem N-(C₁₋₄-Alkyl)pyrrolidin, einem N-(C₁₋₄-Alkyl)piperidin, einem N-(C₁₋₄-Alkyl)morpholin oder einem N-(C₁₋₄-Alkyl)piperazin besteht.

3. Verfahren nach Anspruch 2, wobei die nicht-wäßrige Base aus Pyridin oder Triethylamin besteht.

4. Verfahren nach Anspruch 1, wobei die nicht-wäßrige Base Bicarbonat- oder Carbonationen umfaßt.

5. Verfahren nach einem der vorhergehenden Ansprüche, wobei die wäßrige Base ein Hydroxid oder C₁₋₄-Alkoxid umfaßt.

6. Verfahren nach einem der vorhergehenden Ansprüche, wobei die Säure aus Schwefel-, Chlorwasserstoff-, Phosphor-, Salpeter-, Trifluoressig- und Perchlorsäure ausgewählt ist.

7. Verfahren nach Anspruch 6, wobei die Säure aus Schwefel- oder Chlorwasserstoffsäure besteht.

8. Verfahren nach einem der vorhergehenden Ansprüche, wobei die Thiazolessigsäure aus 2-(Tritylamino)-α-(methoxyimino)-4-thiazolessigsäure besteht.

## Revendications

1. Procédé de préparation d'un acide thiazoleacétique de formule (IV) 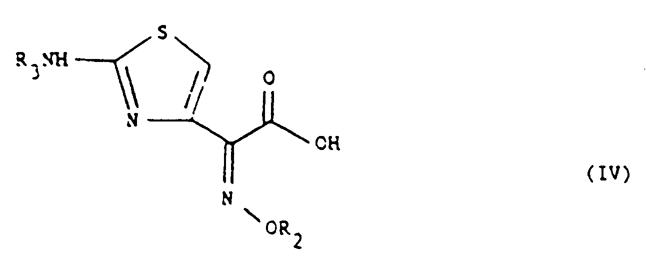 dans laquelle R₂ représente -H,
un groupe alkyle en C₁ à C₄,
un groupe -CO-NH-CH₃,
un groupe -CH₂-CO-CH₂,
un groupe 1,2,4-triazol-5-ylméthyle,
un groupe 1,2,3-triazol-5-ylméthyle,
un groupe -CH₂-CO-OCH₃,
un groupe -CH₂-COOH,
un groupe -C(R₂₋₁)(R₂₋₂)-CO-O-R₂₋₃ dans lequel R₂₋₁ et R₂₋₂ représentent -H ou des groupes -CH₃ et R₂₋₃ représente un groupe alkyle en C₁ à C₄, ou
un groupe -C(R₂₋₁)(R₂₋₂)-COOH dans lequel R₂₋₁ et R₂₋₂ répondent aux définitions précitées ; et
R₃ représente un groupe φ₃C- ou Cl₂CH-CO-, qui comprend
(1) la mise en contact d'un aminothiazole de formule (I) 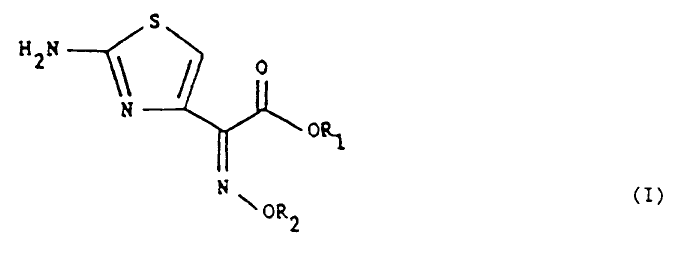 dans laquelle R₁ représente un groupe alkyle en C₁ à C₄, benzyle ou p-méthoxybenzyle et R₂ répond à la définition précitée, avec un composé de formule R₃-X₁ dans laquelle X₁ représente un groupe -Cl ou -Br et R₃ répond à la définition précitée, en présence d'une base non aqueuse,
(2) l'hydrolyse du produit de l'étape (1) avec une base aqueuse, en présence d'un solvant pour le produit d'hydrolyse, et
(3) une acidification à un pH < 7, sans isolement de n'importe quel intermédiaire.

2. Procédé suivant la revendication 1, dans lequel la base non aqueuse est la pyridine, facultativement substituée avec 1 à 3 groupes CH₃, le DBU, le DBN, une tri(alkyle en C₁ à C₄)-amine, une N-(alkyle en C₁ à C₄)-pyrrolidine, une N-(alkyle en C₁ à C₄)-pipéridine, une N-(alkyle en C₁ à C₄)-morpholine ou une N-(alkyle en C₁ à C₄)-pipérazine.

3. Procédé suivant la revendication 2, dans lequel la base non aqueuse est la pyridine ou la triéthylamine.

4. Procédé suivant la revendication 1, dans lequel la base non aqueuse comprend l'ion bicarbonate ou l'ion carbonate.

5. Procédé suviant l'une quelconque des revendications précédentes, dans lequel la base aqueuse comprend un hydroxyde ou un alcoolate en C₁ à C₄.

6. Procédé suivant l'une quelconque des revendications précédentes, dans lequel l'acide est choisi entre les acides sulfurique, chlorhydrique, phosphorique, nitrique, trifluoracétique et perchlorique.

7. Procédé suivant la revendication 6, dans lequel l'acide est l'acide sulfurique ou l'acide chlorhydrique.

8. Procédé suivant l'une quelconque des revendications précédentes, dans lequel l'acide thiazole-acétique est l'acide 2-(tritylamino)-α-(méthoxyimino)-4-thiazoleacétique.
